Europäisches Patentamt

European Patent Office

Office européen des brevets

(19)

(11) Veröffentlichungsnummer : **0 260 286 B1**

(12) **EUROPÄISCHE PATENTSCHRIFT**

(45) Veröffentlichungstag der Patentschrift :
**22.01.92 Patentblatt 92/04**

(51) Int. Cl.⁵ : **A61F 9/00**

(21) Anmeldenummer : **87901385.2**

(22) Anmeldetag : **09.03.87**

(86) Internationale Anmeldenummer :
**PCT/DE87/00102**

(87) Internationale Veröffentlichungsnummer :
**WO 87/05204 11.09.87 Gazette 87/20**

(54) **SPALTLAMPENGERÄT ZUR LASERBEHANDLUNG DES AUGES.**

(30) Priorität : **08.03.86 DE 3607721**

(43) Veröffentlichungstag der Anmeldung :
**23.03.88 Patentblatt 88/12**

(45) Bekanntmachung des Hinweises auf die
Patenterteilung :
**22.01.92 Patentblatt 92/04**

(84) Benannte Vertragsstaaten :
**AT CH DE FR GB IT LI**

(56) Entgegenhaltungen :
**EP-A- 0 139 941
EP-A- 0 140 639
EP-A- 0 191 688
DE-A- 3 148 748
DE-A- 3 306 981
DE-A- 3 425 975
JP-A-57 208 524
US-A- 4 331 132
US-A- 4 443 075**

(73) Patentinhaber : **G. RODENSTOCK
INSTRUMENTE GMBH
Drachenseestrasse 10-12
W-8000 München 70 (DE)**

(72) Erfinder : **FEUERSTEIN, Manfred
Gottfried-Böhm-Ring 23
W-8000 München 70 (DE)**
Erfinder : **KLINGBEIL, Ulrich
Daglfingerstr. 108
W-8000 München 81 (DE)**
Erfinder : **LANGOSCH, Herbert
Thalkirchnerstr. 282
W-8000 München (DE)**
Erfinder : **REIS, Werner
Hilblestr. 40
W-8000 München 19 (DE)**
Erfinder : **WILMS, Karl-Heinz
Hans-Bierlingstr. 47
W-8080 Emmering (DE)**
Erfinder : **EISENTRÄGER, Wolfgang
Prätoriusweg 6
W-8000 München 83 (DE)**
Erfinder : **BISLE, Werner
Karwendelstr. 21
W-8027 Neuried (DE)**

(74) Vertreter : **Münich, Wilhelm, Dr. et al
Kanzlei Münich, Steinmann, Schiller
Willibaldstrasse 36/38
W-8000 München 21 (DE)**

**Beschreibung**

**Technisches Gebiet**

Die Erfindung bezieht sich auf ein Spaltlampengerät zur Laserbehandlung des Auges gemäß dem Oberbegriff des Patentanspruchs 1.

**Stand der Technik**

Ein Spaltlampengerät gemäß dem Oberbegriff des Patentanspruchs 1 ist beispielsweise aus der DE-A-34 25 975 bekannt. Bei diesem Spaltlampengerät ist eine Projektionseinrichtung vorgesehen, die ein zuvor aufgenommenes Bild und insbesondere ein Angiogramm des zu behandelnden Auges in den Beobachtungsstrahlengang einspiegelt.

Bei dem aus der DE-A-34 25 975 bekannten Spaltlampengerät muß die Bedienungsperson nicht nur das eingespiegelte Bild mit dem tatsächlich beobachteten Bild des zu behandelnden Auges zur Deckung bringen, sondern auch laufend den Laser "nachführen", wenn sich das Auge der zu behandelnden Person auch nur geringfügig bewegt hat.

Es ist ohne weiteres einzusehen, daß die Bedienungsperson durch die beiden Vorgänge - Nachführen des Beobachtungsstrahlengangs beispielsweise einer Spaltlampe, um das Angiogramm mit dem Fundusbild in Deckung zu bringen, Nachführen des Lasers auf die zu koagulierende Stelle - stark belastet ist. Dies gilt insbesondere dann, wenn der Strahl des Behandlungslasers nicht koaxial mit dem Beobachtungsstrahlengang geführt ist. Eine derartige Belastung kann unter Umständen zu Fehlbedienungen und damit zu nachteiligen Behandlungsergebnissen führen.

Es ist deshalb bereits eine Vorrichtung anderer Gattung, nämlich eine Vorrichtung, bei der ein indirektes Ophthalmoskop verwendet wird, vorgeschlagen worden, bei der eine Steuereinheit vorgesehen ist, die den Laserstrahl bei Bewegungen des Auges nachführt (ARVO-Abstracts, Band 38, Zusammenfassung Nr. 92). Bei dieser bekannten Vorrichtung ist eine Bildverarbeitungseinheit vorgesehen, die ein von einer Bilderfassungseinrichtung während der Behandlung geliefertes Bild analysiert. Das Nachführen erfolgt bei dieser bekannten Vorrichtung dergestalt, daß die Bedienungsperson einen markanten Bereich, beispielsweise eine Blutgefäßverzweigung auswählt, und die Steuereinheit den Laserstrahl so nachführt, daß die Abstandsbeziehung der zu behandelnden Stelle zu diesem markanten Bereich während der Behandlung konstant bleibt. Der Grund für diese Vorgehensweise ist, daß die gegenwärtig zu in der ärzrlichen Praxis tragbaren Gestehungskosten realisierbaren Bildverarbeitungseinheiten nicht in der Lage sind, beispielsweise den gesamten Augenhintergrund in "Echtzeit" zu analysieren, was zur Realisierung des Nachführens unter Berücksichtigung des gesamten erfaßten Bereichs erforderlich wäre.

Bei dieser bekannten Vorrichtung wird jedoch nicht berücksichtigt, daß es in einem menschlichen Auge im Rahmen der Auflösung einer typischen Bildverarbeitungseinheit "morphologisch" ähnliche Bereiche, beispielsweise Blutgefäßverzweigungen geben kann.

Bei einer schnellen Augenbewegung ist es deshalb möglich, daß die Steuereinheit eine andere "morphologisch ähnliche" Blutgefäßverzweigung als den anfänglich ausgewählten Bereich erfaßt und diesen Bereich fälschlich als den Bereich identifiziert, zu dem die Abstandsbeziehung des Lasers konstant gehalten werden soll. Tritt dieser Fall auf, so richtet die Steuereinheit den Laser auf einen anderen Bereich als den zu behandelnden Bereich aus. Es bedarf keiner näheren Erläuterung, daß es dann bei dieser bekannten Vorrichtung zu unannehmbaren Schädigungen des Auges kommen kann.

**Darstellung der Erfindung**

Der Erfindung liegt die Aufgabe zugrunde, ein Spaltlampengerät gemäß dem Oberbegriff des Patentanspruchs 1 derart weiterzubilden, daß einerseits die Bedienung erleichtert und andererseits Fehlerquellen durch fehlerhafte Ausrichtung des Lasers etc. ausgeschlossen werden.

Eine erfindungsgemäße Lösung dieser Aufgabe ist mit ihren Weiterbildungen in den Patentansprüchen gekennzeichnet.

Erfindungsgemäß ist erkannt worden, daß eine Ursache für Fehler bei Laserbehandlungen und insbesondere bei Laserkoagulationen ist, daß die Bedienungsperson bei der Behandlung, bei der sie aus einer Reihe von Gründen unter Zeitdruck steht, die zu behandelnde Stelle, die sie anhand bestimmter Strukturen wiederfinden soll, mit einem Bereich ähnlicher Struktur verwechselt.

Erfindungsgemäß sind deshalb in dem zuvor aufgenommenen Bild des Auges die zu behandelnde Stelle bzw. Stellen markiert. Ein Bildsensor erfaßt das zu behandelnde Auge.

Die Steuereinheit richtet aufgrund des Ausgangssignals des Bildsensors den Laser bei Bewegungen des Auges auf die in dem überlagerten Bild markierte Stelle aus.

Durch die erfindungsgemäß vorgesehene Markierungen können Fehler, die beispielsweise durch das falsche Identifizieren von Strukturen hervorgerufen werden, vermieden werden.

Als weitere Hilfestellung für die Bedienungsperson positioniert die Steuereinheit den Laserstrahl so vor, daß er auf die markierte Stellung gerichtet ist.

Der Bildsensor, der das zu behandelnde Auge detektiert, und der z.B. eine Fernsehkamera sein kann, kann auch dazu verwendet werden, das zu behandelnde Auge auf einem Kontrollmonitor darzustellen.

Durch die Kombination dieser Merkmale wird erreicht, daß der behandelnde Arzt in Ruhe vor Beginn der eigentlichen Behandlung einen Operationsplan erstellen kann, in dem er sich die zu behandelnden, beispielsweise die zu koagulierenden Stellen auf einem Bild des zu behandelnden Auges sucht und diese markiert. In dem Teil der Behandlung, in dem der Patient auf dem Operationsstuhl sitzt, und in dem erfahrungsgemäß schnell gearbeitet werden muß, positioniert die erfindungsgemäß vorgesehene Steuereinheit den Laserstrahl für den Arzt so vor, daß er sich nur noch von der Güte der Vorpositionierung überzeugen, eventuell notwendige Korrekturen durchführen und anschließend den Laserstrahl auslösen muß.

Andererseits wird durch die erfindungsgemäße Ausbildung erreicht, daß die Bedienungsperson, also der behandelnde Arzt jederzeit die Güte der Positionierung des Laserstrahls anhand des eingespiegelten Bildes, das beispielsweise den gesamten Augenhintergrund wiedergibt, überprüfen kann. Da die Bedienungsperson die Übereinstimmung des eingespiegelten Bildes mit dem tatsächlichen Bild über einen großen Bereich des Auges "visuell" überprüfen kann, können "Laser-Fehlschüsse", die dadurch entstehen, daß fälschlicherweise zwei ähnlich aussehende Bereiche miteinander zur Deckung gebracht sind, nicht auftreten. Trotzdem ist bei der erfindungsgemäßen Vorrichtung lediglich ein geringer Rechenaufwand erforderlich, da beispielsweise nicht der gesamte, sehr stark strukturierte Augenhintergrund überprüft werden muß, sondern nur der Laserstrahl einer Markierung nachgeführt werden muß.

Selbstverständlich ist es auch möglich, den Laserstrahl zusätzlich manuell auszurichten (Anspruch 13), so daß der Arzt die Wahlfreiheit bei der Behandlung behält.

Mit der erfindungsgemäßen Spaltlampengerät ist es damit möglich, den Arzt von zeitaufwendigen und häufig von Hand nur ungenau durchzuführenden Vorgängen zu entlasten, ohne ihm die ärztliche Verantwortung bei der eigentlichen Behandlung zu nehmen.

Die erfindungsgemäße Spaltlampengerät ist in vorteilhafter Weise sowohl bei der Behandlung des Fundus als auch bei der Behandlung vorderer Augenabschnitte, wie der Cornea einsetzbar, und eignet sich zur Verwendung mit Behandlungslasern aller Wellenlängenbereiche, die im sichtbaren, im UV- oder im Infrarotbereich arbeiten. Auch ist es ohne weiteres möglich, mit einem gesonderten Ziellaser zu arbeiten.

Weiterbildungen der Erfindung sind in den Unteransprüchen gekennzeichnet.

Die Ausgangssignale des Bildsensors können auch in eine Bildverarbeitungseinrichtung (Anspruch 2) eingegeben werden und so verschiedene Steuer- bzw. Regelvorgänge auslösen:

Beispielsweise kann gemäß Anspruch 3 die Steuereinheit das vorpositionierte eingespiegelte Bild, das die Bedienungsperson mit dem tatsächlichen Bild des zu behandelnden Auges, beispielsweise dem Fundus, zur Deckung gebracht hat, bei Bewegungen des Auges nachführen. Damit muß die Bedienungsperson zu einer Behandlung nur noch zu Beginn den Beobachtungsstrahlengang so justieren, daß der Beobachtungsstrahlengang gleich mit dem aufgenommenen Bild verläuft. Hierdurch ergibt sich eine weitere Bedienungsvereinfachung, da die Bedienungsperson nur noch zu Beginn der Behandlung dafür sorgen muß, daß das eingespiegelte Bild mit der Vorlage des Auges zur Deckung gebracht ist:

Der Nachführgang kann dabei in der Weise erfolgen, wie dies beispielsweise gemäß der eingangs genannten Literaturstelle ARVo-Abstracts der Fall ist. Trotzdem hat man bei der erfindungsgemäßen Vorrichtung den Vorteil, daß der Arzt aufgrund des eingespiegelten Bildes sofort erkennt, wenn die Steuereinheit eine falsche Stelle als Referenz-Bereich identifiziert; damit kann der Arzt die Vorrichtung abschalten bzw. korrigieren.

Das eingespiegelte Bild kann beispielsweise in der in der DE-A-34 25 975 beschriebenen Weise eingespiegelt und zur Deckung gebracht werden: Beispielsweise kann ein Diapositiv mittels einer Variooptik in den Beobachtungsstrahlengang eingespiegelt werden. Durch Verschieben des Diapositivs und Ändern der Vergrößerung durch Verstellen der Variooptik kann erreicht werden, daß sich das eingespiegelte Bild mit der Vorlage deckt. Diese Vorgänge kann die Steuereinheit beispielsweise über Stellmotore ausführen.

Besonders vorteilhaft ist es jedoch, wenn das eingespiegelte Bild ein videobild ist (Anspruch 4), da sich dieses Videobild leicht mittels Bildverarbeitung bearbeiten läßt, also beispielsweise die zu behandelnde Stelle leicht an einem entsprechenden Bildverarbeitungs-Eingabegerät markiert werden kann. Darüberhinaus kann das videobild im Beobachtungsstrahlengang leicht beispielsweise mittels Hardware- oder Softwarescrolling nachgeführt und in der Größe angepaßt werden, ohne daß mechanische Verschiebungen von Optikelementen erforderlich wären.

Um auch größere Bewegungen des Auges ausgleichen zu können, ist es gemäß Anspruch 3 auch möglich, daß die Steuereinheit den Beobachtungsstrahlengang, also beispielsweise das Spaltlampenmikroskop mit dem eingespiegelten Bild verfährt.

Gemäß den Ansprüchen 6 und 7 weist die erfindungsgemäß vorgesehene Steuereinheit einen Speicher auf, in dem die Behandlungsparameter, wie Ort der Koagulation oder des Schnitts in der Cornea, Leistung des Laserstrahls, Schußzeit etc. speicherbar sind. Diese Speicherung der Behandlungsparameter erlaubt nicht nur eine lückenlose Dokumentation, sondern ermöglicht auch eine Auswertung der mit der erfindungsgemäßen Spaltlampengerät durchgeführten Behandlungsvorgänge in wissenschaftlicher und/oder rechtlicher Hinsicht.

Dabei ist es in jedem Falle vorteilhaft, wenn die Steuereinheit ein Ausgabegerät, beispielsweise einen Protokolldrucker aufweist, mit dem die Soll- und Ist-Behandlungsparameter ausgegeben werden (Anspruch 8).

Besonders vorteilhaft ist es, wenn sowohl die für die Behandlung vorgegebenen Parameter als auch die tatsächlichen Behandlungsparameter im Beobachtungsstrahlengang erkennbar sind (Anspruch 8), wobei es insbesondere vorteilhaft ist, wenn eine Zuordnung der Parameter zu der jeweiligen Markierung erfolgt (Anspruch 9).

Die Steuereinheit kann in Falle auch dazu verwendet werden, verschiedene Sicherungsfunktionen auszuführen:

Gemäß Anspruch 10 ist vorgesehen, daß die Steuereinheit den Behandlungsvorgang unterbricht, wenn das projizierte Bild nicht mehr ausreichend mit dem Auge in Deckung ist.

Eine Abschaltung des Laserstrahls kann auch erfolgen, wenn die Weißverfärbung des Fundus eine bestimmte Stufe erreicht hat (Anspruch 11). Darüberhinaus kann die Steuereinheit auch die vom Laser deponierte Energie regeln (Anspruch 12).

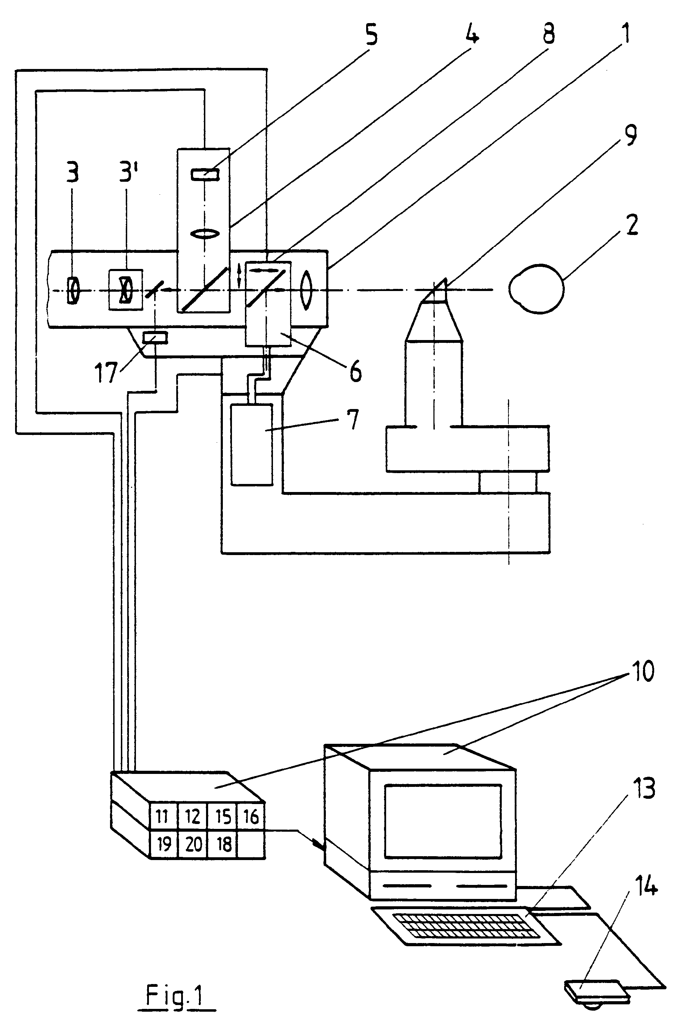

## Kurze Beschreibung der Zeichnung

Die Erfindung wird nachstehend anhand eines Ausführungsbeispiels unter Bezugnahme auf die Zeichnung näher beschrieben,

deren einzige Figur schematisch ein erfindungsgemäßes Spaltlampengerät zeigt.

## Darstellung eines Ausführungsbeispiels

Das erfindungsgemäße Spaltlampengerät weist in an sich bekannter Weise ein Beobachtungsmikroskop (1) beispielsweise eines Spaltlampengeräts auf, mit dem ein zu untersuchendes und zu behandelndes Auge (2) betrachtet werden kann. In dem Beobachtungsmikroskop (1) sind ein Vergrößerungswechsler (3'), ein Okularsystem (3) und eine Projektionseinrichtung (4) für ein Referenzbild vorgesehen. Die Projektionseinrichtung (4) für die Referenzbild-Einspiegelung weist bei dem gezeigten Ausführungsbeispiel einen Videomonitor (5) auf. Ferner ist eine Einspiegeleinrichtung (6) für einen Laser (7) mit einem Strahlmanipulator (8) vorgesehen, der es erlaubt, den eingespiegelten Laserstrahl auf eine bestimmte Stelle des zu behandelnden Auges auszurichten. Mit (9) ist die Spaltleuchte des Spaltlampengeräts bezeichnet.

Die nur schematisch dargestellte Steuereinheit (10) weist einen zur Bildverarbeitung geeigneten Rechner (11), einen Steuermonitor (12), eine Tastatur (13), die gegebenenfalls auch eine Maus-Eingabe (14) aufweisen kann, ein Anzeigepanel (15), eine Bildspeichereinheit (16), einen Bildsensor (17), der das zu behandelnde Auge (2) detektiert, und einen Bildmonitor (18) sowie einen externen Speicher (19) beispielsweise ein Diskettenlaufwerk und einen Drukker (20) auf.

Die Steuereinheit (10) steuert nicht nur die Leistung und die Schußzeit des Lasers, sondern auch über den Strahlmanipulator (8) den Auftreffort des Laserstrahls im Auge derart, daß er mit einer im eingespiegelten Bild vorgesehenen Markierung übereinstimmt. Ferner führt die Steuereinheit (10) das eingespiegelte Bild, das die Bedienungsperson mit dem Auge zur Deckung gebracht hat, bei Bewegungen des zu untersuchenden Auges nach. Die Steuereinheit erlaubt ferner die Manipulation eines zuvor mit dieser Spaltlampengerät oder mit einem anderen Untersuchungsgerät aufgenommenen bildes vor dem eigentlichen Behandlungsvorgang. Damit kann beispielsweise ein Chefarzt die Behandlung - Koagulations- bzw. Schnittort, deponierte Energie etc. - planen, die eigentliche Behandlung aber einem Assistenten überlassen.

Vorstehend ist die Erfindung anhand eines Ausführungsbeispiels beschrieben worden. Innerhalb des allgemeinen Erfindungsgedankens sind natürlich die verschiedensten Modifikationen möglich: Beispielsweise ist anstelle eines Spaltlampengeräts auch die Verwendung eines anderen Untersuchungs- und Beobachtungsgeräts möglich. Ferner kann die Steuereinheit auch die Regelung anderer Größen übernehmen, z.B. kann sie die deponierte Energie gemäß den in der DE-A-30 24 169 oder der DE-A-33 06 981 beschriebenen Algorithmen regeln.

**Patentansprüche**

1. Spaltlampengerät zur Laserbehandlung des Auges mit
   – einer Projektionseinrichtung (4), die in einen Beobachtungsstrahlengang (1) ein Bild des zu behandelnden Auges (2) einspiegelt, das von einer Bedienungsperson manuell mit dem Bild des zu behandelnden Auges zur Deckung gebracht wird,
   – einem Laser (7), dessen Strahl an die zu behandelnde Stelle des Auges führbar ist, und
   – einer elektronischen Steuereinheit (10), die unter anderem die vom Laser deponierte Energie steuert,
dadurch **gekennzeichnet**, daß in dem zuvor aufgenommenen Bild des Auges die zu behandelnde Stelle bzw. Stellen markiert sind,
daß ein Bildsensor (17) das zu behandelnde Auge (2) erfaßt, und
daß die Steuereinheit (10) aufgrund des Ausgangssignals des Bildsensors den Laser (7) bei Bewegungen des Auges auf die in dem überlagerten Bild markierte Stelle ausrichtet.

2. Spaltlampengerät nach Anspruch 1,
dadurch **gekennzeichnet**, daß die Steuereinheit eine Bildverarbeitungseinrichtung (11) aufweist.

3. Spaltlampengerät nach Anspruch 1 oder 2,
dadurch **gekennzeichnet**, daß die Steuereinheit den Beobachtungsstrahlengang mit dem eingespiegelten Bild, das die Bedienungsperson manuell mit dem zu behandelnden Auge zur Deckung gebracht hat, bei Bewegungen des Auges nachführt.

4. Spaltlampengerät nach einem der Ansprüche 1 bis 3,
dadurch **gekennzeichnet**, daß das eingespiegelte Bild ein Videobild ist.

5. Spaltlampengerät nach einem der Ansprüche 1 bis 4,
dadurch **gekennzeichnet**, daß die Steuereinheit einen Speicher aufweist, in dem die Parameter für die Behandlung speicherbar sind.

6. Spaltlampengerät nach Anspruch 5,
dadurch **gekennzeichnet**, daß in dem Speicher die tatsächlichen Behandlungsparameter speicherbar sind.

7. Spaltlampengerät nach Anspruch 5 oder 6,
dadurch **gekennzeichnet**, daß die Steuereinheit eine Ausgabeeinheit (20) aufweist.

8. Spaltlampengerät nach Anspruch 5, 6 oder 7,
dadurch **gekennzeichnet**, daß die Soll- und/oder Ist-Behandlungsparameter in den Beobachtungsstrahlengang eingespiegelt sind.

9. Spaltlampengerät nach einem der Ansprüche 1 bis 8,
dadurch **gekennzeichnet**, daß die Behandlungsparameter aus der Markierung im eingespiegelten Bild erkennbar sind.

10. Spaltlampengerät nach einem der Ansprüche 1 bis 9,
dadurch **gekennzeichnet**, daß die Steuereinheit die Behandlung unterbricht, wenn das projizierte Bild ungenügend mit dem Auge zur Deckung gebracht ist.

11. Spaltlampengerät nach einem der Ansprüche 1 bis 10,
dadurch **gekennzeichnet**, daß die Steuerschaltung einen Detektor aufweist, der die Weißverfärbung des Koagulats erfaßt.

12. Spaltlampengerät nach Anspruch 11,
dadurch **gekennzeichnet**, daß die Steuerschaltung die vom Laser deponierte Energie regelt.

13. Spaltlampengerät nach einem der Ansprüche 1 bis 15,
dadurch **gekennzeichnet**, daß der Laserstrahl zusätzlich manuell ausrichtbar ist.

**Claims**

1. A slit lamp unit for the laser treatment of the eye with
   – a projection device (4) which images into an observation beam path (1) an image of the eye to be treated (2) which is made flush with the image of the eye to be treated manually by the operator,
   – a laser (7) whose beam can be directed to the portion of the eye to be treated, and
   – an electronic control unit (10) which, among other things, controls the energy deposited by the laser,
characterized by the fact that the portion or portions to be treated are marked in the previously taken image of the eye,
that an image sensor (17) registers the eye to be treated (2), and
that the control unit (10) aligns the laser (7) onto the portion marked in the superimposed image when the eye is moved on the basis of the output signal of the image sensor.

2. A slit lamp unit in accordance with claim 1,
characterized by the fact that the control unit possesses an image processing device (11).

3. A slit lamp unit in accordance with claims 1 or 2,
characterized by the fact that the control unit realigns the observation beam path with the imaged image which the operator has manually made flush with the eye to be treated when the eye moves.

4. A slit lamp unit according to any of claims 1 to 3,
characterized by the fact that the imaged image is a video image.

5. A slit lamp unit according to any of claims 1 to 4,
characterized by the fact that the control unit possesses a memory in which the parameters for the treatment can be stored.

6. A slit lamp unit in accordance with claim 5,
characterized by the fact that the actual treatment parameters can be stored in the memory.

7. A slit lamp unit in accordance with claims 5 or 6,
characterized by the fact that the control unit possesses an output unit (20).

8. A slit lamp unit according to any of claims 5, 6 or 7,
characterized by the fact that the nominal and/or actual treatment parameters are imaged into the observation beam path.

9. A slit lamp unit according to any of claims 1 to 8,
characterized by the fact that the treatment parameters can be seen from the markings in the imaged image.

10. A slit lamp unit according to any of claims 1 to 9,
characterized by the fact that the control unit interrupts the treatment whenever the projected image is not sufficiently flush with the eye.

11. A slit lamp unit according to any of claims 1 to 10,
characterized by the fact that the control circuit possesses a detector which registers the white discoloration of the coagulate.

12. A slit lamp unit in accordance with claim 11,
characterized by the fact that the control circuit regulates the energy deposited by the laser.

13. A slit lamp unit according to any of claims 1 to 12,
characterized by the fact that the laser beam can also be aligned manually.

## Revendications

1. Appareil à lampe à fente destiné au traitement par laser de l'oeil, cet appareil comportant
– un dispositif (4) de projection, qui envoie dans le chemin optique (1) du rayon d'observation une image de l'oeil (2) qu'il s'agit de traiter, laquelle image est amenée manuellement par l'opérateur en coïncidence avec l'image de l'oeil qu'il s'agit de traiter,
– un laser (7) dont le rayon peut être guidé sur l'emplacement qu'il s'agit de traiter dans l'oeil,
– et une unité de commande électronique (10) qui commande, notamment, l'énergie fournie par le laser, appareil
caractérisé en ce que, dans l'image de l'oeil prise précédemment, le ou les emplacements à traiter sont marqués
en ce qu'un capteur (17) d'image saisit l'oeil (2) qu'il s'agit de traiter et
en ce que l'unité (10) de commande oriente le laser, dans le cas de mouvements de l'oeil, en fonction du signal de sortie du capteur d'image, sur l'emplacement marqué sur l'image superposée.

2. Appareil à lampe à fente selon la revendication 1,
caractérisé en ce que l'unité de commande présente un dispositif (11) de traitement d'image.

3. Appareil selon la revendication 1 ou 2,
caractérisé en ce que, dans le cas où l'oeil bouge, l'unité de commande corrige le chemin du rayon d'observation au moyen de l'image projetée que l'opérateur a amenée manuellement en correspondance avec l'oeil à traiter.

4. Appareil à lampe à fente selon une des revendications 1 à 3,
caractérisé en ce que l'image projetée est une image vidéo.

5. Appareil à lampe à fente selon une des revendications 1 à 4,
caractérisé en ce que l'unité de commande présente une mémoire dans laquelle on peut mettre en mémoire les paramètres du traitement.

6. Appareil à lampe à fente selon la revendication 5,
caractérisé en ce que les paramètres réels du traitement peuvent être mis en mémoire.

7. Appareil à lampe à fente selon la revendication 5 ou 6,
caractérisé en ce que l'unité de commande présente une unité d'édition (20) (ou imprimante (20)).

8. Appareil à lampe à fente selon les revendications 5, 6 ou 7,
caractérisé en ce que les paramètres prescrits et/ou les paramètres réels du traitement sont projetés dans le chemin optique du rayon d'observation.

9. Appareil à lampe à fente selon une des revendications 1 à 8,
caractérisé en ce que les paramètres de traitement peuvent être identifiés à partir du marquage présent dans l'image projetée.

10. Appareil à lampe à fente selon une des revendications 1 à 9,
caractérisé en ce que l'unité de commande interrompt le traitement si l'image projetée n'est pas mise suffisamment en coïncidence avec l'oeil.

11. Appareil à lampe à fente selon une des revendications 1 à 10,
caractérisé en ce que le circuit de commande présente un détecteur qui capte la coloration en blanc du coagulat.

12. Appareil à lampe à fente selon la revendication 11,
caractérisé en ce que le circuit de commande régule l'énergie fournie par le laser.

13. Appareil à lampe à fente selon une des revendications 1 à 12,
caractérisé en ce que le rayon laser peut en outre être orienté manuellement.

Fig.1